Europäisches Patentamt

(19) European Patent Office

Office européen des brevets

(11) **EP 0 804 925 A1**

(12) **EUROPEAN PATENT APPLICATION**
published in accordance with Art. 158(3) EPC

(43) Date of publication:
05.11.1997 Bulletin 1997/45

(51) Int. Cl.⁶: **A61K 9/127**, A61K 47/24,
B01J 13/02

(21) Application number: 95913349.7

(22) Date of filing: 27.03.1995

(86) International application number:
PCT/JP95/00566

(87) International publication number:
WO 95/26185 (05.10.1995 Gazette 1995/42)

(84) Designated Contracting States:
AT BE CH DE DK ES FR GB GR IE IT LI LU MC NL PT SE
Designated Extension States:
LT SI

(30) Priority: 28.03.1994 JP 57409/94

(71) Applicant: DAIICHI PHARMACEUTICAL CO., LTD.
Chuo-ku, Tokyo 103 (JP)

(72) Inventors:
- KIKUCHI, Hiroshi,
  Daiichi Pharmaceutical Co., Ltd.
  Edogawa-ku, Tokyo 134 (JP)
- YACHI, Kiyoto,
  Daiichi Pharmaceutical Co., Ltd.
  Edogawa-ku, Tokyo 134 (JP)

(74) Representative: Kinzebach, Werner, Dr. et al
Patentanwälte
Reitstötter, Kinzebach und Partner
Postfach 86 06 49
81633 München (DE)

(54) **LIPOSOME WITH INCREASED RETENTION VOLUME**

(57)    The present invention provides liposome preparations capable of efficiently holding water soluble substance particularly non-electrolytic water soluble substances.

Liposomes and suspensions thereof comprising multilamellar liposomes characterized in that the liposome membrane is formed from a neutral phospholipid comprising a substantially saturated fatty acid residue a charged phospholipid comprising a substantially saturated fatty acid residue, ratio of said neutral phospholipid and charged phospholipid is 200:1 to 3:1, weighted average particle size of the liposome is 50 nm to 3,000 nm, and an encapsulation capacity of the liposome (internal aqueous phase (volume)/lipids (weight)) is at least 5 mℓ/g.

The present liposome preparation can encapsulate an aqueous solution of a non-electrolytic water soluble substances with an encapsulation capacity (internal aqueous phase/lipids) of at least 5 mℓ/g.

EP 0 804 925 A1

## Description

Technical Field

The present invention relates to novel liposome preparations, and suspension thereof as well as a process for production thereof and particularly to liposomes and suspension thereof capable of holding a large amount of a water soluble non-electrolytic drug.

Related Art

Liposomes are vesicles comprising unilamellar or multilamellar lipid membranes which encapsulate aqueous phase (designated as an internal aqueous phase), and since they can maintain drugs within the lipid membranes separatedly from the outer aqueous phase, it has been enthusiastically researched as a drug delivery system. Liposomes can encapsulate various kind of drugs including high molecular weight drugs, i.e., both lipid-soluble drugs and water-soluble drugs.

A lipid-soluble drug is easily encapsulated in liposomes due to its affinity with the lipid membrane of the liposome. On the other hand, among water soluble drugs, water soluble electrolytic drugs are effectively encapsulated in the internal aqueous phase in liposomes by electrostatic interaction between the charge of the drug and oppositely charged phospholipid thereto (Japanese Patent Application No. 2-187370), or by pH gradient between the outside phase and the inside phase of liposomes (WPI 88-271022/38).

However, the amount of encapsulated drug has still not been satisfactory. Further, if such electrostatic interaction method is used, drugs are limited to drugs oppositely charged to the "charged" phospholipid, and if a drug having the same charge as that of phospholipid is used, an encapsulation ratio of the used drug into liposomes is very low. Moreover, where the drug is a water soluble non-electrolytic substance, the above-mentioned means cannot be applied, and therefore it has been not easy to efficiently encapsulate a water-soluble non-electrolytic drug in liposomes.

As means for efficiently encapsulating a water-soluble non-electrolyte in the internal aqueous phase of liposomes, reverse-phase evaporation method (Proc. Natl. Acad. Sci. USA, Vol. 75(9), p4194, 1978), ether injection method (ibid, Vol. 443, p629, 1975) and the like were proposed. However, since such methods use an ether having a low ignition point, these methods cannot be used for industrial production of a large amount of liposomes. Moreover, liposomes produced by these methods are cominantly unilamellar liposomes. Unilamellar liposomes have a drawback in that their stability is low in the blood, after being administered, in comparison with multilamellar liposomes.

Further WO88/09165 discloses liposomes encapsulating water-soluble non-electrolytic drugs, however, the encapsulation-ratio is very low.

Disclosure of Invention

Accordingly, the present invention provides a liposome preparations comprising multilamellar liposomes, capable of encapsulating and holding a large amount of water-soluble drugs, especially water-soluble non-electrolytic drugs or drugs having the same charge as that of phospholipid.

The present inventors, as a result of various researches, completed the present invention by finding that liposomes capable of holding a large amount of water in the internal aqueous phase can be formed by using a neutral phospholipid satisfying a predetermined condition and a charged phospholipid satisfying a predetermined condition in a particular ratio.

Accordingly, the present invention provides liposome preparations comprising multilamellar liposomes characterized in that the membranes of liposomes are formed from a neutral phospholipid comprising a substantially saturated fatty acid residue and a charged phospholipid comprising a substantially saturated fatty acid residue, weight ratio of the neutral phospholipid and the charged phospholipid is 200:1 to 3:1, weighted average particle diameter of the liposomes is 50 nm to 3,000 nm, and an encapsulation capacity of the liposomes (internal aqueous phase (volume)/lipids (weight)) is at least 5 m$\ell$/g.

According to the present invention, liposome preparations having a high encapsulation capacity of the internal aqueous phase and therefore capable of holding a large amount of water-soluble drug, particularly water soluble non electrolytic drug, and stable in the blood and during storage are provided.

Best Mode for Carrying Out the Invention

One of the essential components for forming membrane of the present liposomes is a neutral phospholipid comprising a substantially saturated fatty acid residue. The number of carbon atoms in a fatty acid residue in the phospholipid is usually at least 14, preferably at least 15, and more preferably at least 16. Where the number of carbon atoms in a fatty acid residue is less than 14, an ability of liposomes to hold the internal aqueous phase is low and stability of the liposomes in the blood after administration is low. On the other hand, generally, where the number of carbon atoms in a fatty acid residue is 28 or more, biocompatibility becomes low, and very high temperature is necessary during production of liposomes.

The phrase "substantially saturated" means that a fatty acid used is fully saturated (containing no C-C double bonds), or that an extent of the unsaturation of a fatty acid is very low. For a fatty acid residue being substantially saturated, it is necessary that an extent of the unsaturation of whole neutral phospholipid shown by an iodine value is up to 20, and preferably the iodine value is up to 10. Where the extent of the unsaturation is too high, encapsulation ratio is low, and liposomes are easily oxidized and are difficult to heat-sterilize.

The neutral phospholipid used in the present invention is a neutral glycerophospholipid, for example, partially or fully hydrogenated naturally occurring, for example soybean or egg yolk-derived, or synthetic phosphatidylcholine, particularly semi-synthetic or synthetic dipalmitoyl phosphatidylcholine (DPPC) or distearoyl phosphatidylcholine (DSPC).

Another one of the essential components for forming membrane of the present liposomes is a charged phospholipid comprising a substantially saturated fatty acid residue. The number of carbon atoms in a fatty acid residue is usually at least 14, preferably at least 15, and more preferably at least 16. Where the number of carbon atoms in a fatty acid residue is less than 14, an ability of liposomes to hold the internal aqueous phase is low and stability of the liposomes in the blood after administration is low.

On the other hand, where the number of carbon atoms in a fatty acid residue is 28 or more, biocompatibility becomes low, and very high temperature is necessary during production of liposomes. The phrase "substantially saturated" means that a fatty acid used is fully saturated (containing no C-C double bonds), or that an extent of the unsaturation of a fatty acid is very low. For a fatty acid residue being substantially saturated, it is necessary that an extent of the unsaturation of whole charged phospholipid shown by an iodine value is up to 20, and preferably the iodine value is up to 10. Where the extent of the unsaturation is too high, encapsulation ratio is low, and liposomes are easily oxidized and are difficult to be heat-sterilized.

The charged phospholipids used in the present invention are positively or negatively charged glycerophospholipids. The negatively charged phospholipids include, for example, phosphatidylserine, for example, partially or fully hydrogenated naturally occurring, for example, soybean or egg yolk-derived, or semi-synthetic or synthetic phosphatidylserine, particularly semi-synthetic or synthetic dipalmitoyl phosphatidylserine (DPPS) or distearoyl phosphatidylserine (DSPS); phosphatidylglycerol, for example, partially or fully hydrogenated naturally-occurring, for example, soybean or egg yolk-derived, or semisynthetic phosphatidylglycerol, particularly, semi-synthetic or synthetic dipalmitoyl phosphatidylglycerol (DPPG) or distearoyl phosphatidylglycerol (DSPG); phosphatidylinositol, for example, partially or fully hydrogenated naturally occurring, for example soybean or egg yolk-derived, or semi-synthetic or synthetic phosphatidylinositol, particularly semi-synthetic or synthetic dipalmitoyl phosphatidylinositol (DPPI) or distearoyl phosphatidyl-inositol (DSPI); phosphatidic acid, for example, partially or fully hydrogenated naturally occurring, for example soybean or egg yolk-derived, or semi-synthetic or synthetic phosphatidic acid, particularly semi-synthetic or synthetic dipalmitoyl phosphatidic acid (DPPA) or distearoyl phosphatidic acid (DSPA).

Positively charged lipid is, for example, an ester of phosphatidic acid with an aminoalcohol, such as an ester of dipalmitoyl phosphatidic acid or distearoyl phosphatidic acid with hydroxyethylenediamine.

Although such charged phospholipid is used alone, more than one charged phospholipid can be used. In the case where more than one charged phospholipid is used, preferably both the charged phospholipids are positively charged, or both the charged phospholipids are negatively charged in order to prevent aggregation of liposomes.

According to the present invention, a ratio of the neutral phospholipid and the charged phospholipid is 200:1 to 3:1, preferably 60:1 to 4:1, and more preferably 40:1 to 5:1 by weight. Where this ratio is outside of 200:1 to 3:1, encapsulation capacity is low, and it is difficult to obtain the advantages of the present invention.

The present liposomes can contain various optional components in addition to the above-mentioned two essential components. For example, vitamin E ($\alpha$-tocopherol) and/or vitamin E acetate ester as an antioxidant can be added at an amount of 0.01 to 2 molar %, preferably 0.1 to 1 molar % relating to total amount of lipids. Moreover, to control the pharmacokinetics of liposomes after administration, polyglycerol lipid derivatives and/or polyethylene glycol lipid derivatives can be added in an amount of 2 to 10 molar %, preferably 4 to 8 molar % relating a total amount of lipids. By adding these components, the pharmacokinetics can be controlled, thereby capturing of the liposomes by the liver and spleen (reticuloendotherial system) and clearance thereof from the blood can be prevented.

For the present liposomes containing the above-mentioned phospholipids, concentration of total lipid is generally 20 mg/m$\ell$ to 100 mg/m$\ell$, preferably 40 mg/m$\ell$ to 90 mg/m$\ell$, and more preferably 50 mg/m$\ell$ to 80 mg/m$\ell$ in order to enhance to encapsulation of drug in liposome.

The present liposomes hold and encapsulate a drug dissolved in the internal aqueous phase. Such drugs may be water soluble electrolytic type or non-electrolytic type, but the advantages of the present invention is realized especially in encapsulation of a non-electrolytic type. The drugs to be encapsulated in the present liposomes include:

Low molecular weight compound

X-ray imaging agents:

Iodixanol, iohexol, iopamidol, iobazol, iomeprol, ioxylan, iopromide, isovist, iomeprol, iopentol, iobromide, iosymide, ioberuzol, iotrolan, iotasul, iodecymol, 1,3-bis-(N-3,5-bis(2,3-dihydroxypropylaminocarbonyl)-2,4,6-triiodophenyl)-N-hydroxyacetylamino) propane;

MRI imaging agent:

Dimeglumine gadopentetate, gadodiamide, prohance.

Anti-tumor agents:

- Anthracycline type anti-tumor agents such as doxorubicin hydrochloride, daunorubicin hydrochloride, epirubicin
- Camptotecin derivatives
        Irinotecan hydrochloride
- Antimetabolites such as methotrexate, cytocinarabinoside etc.
- Plant alkaloids such as vinblastine sulfate, vinclistine sulfate, taxol, taxotel etc.
- Hormons such as tamoxifen citrate
- Bleomycin derivatives such as bleomycin sulfate etc.
- Actinomycin derivatives such as actinomycin D etc.
- Cisplastin
- Mytomycin C
- Neocartinostatin

Antibiotics:

- Tetracyclines such as tetracycline hydrochloride etc.
- Aminoglycoside antibiotics such as gentamycin sulfate etc.
- Other antibiotics such as streptomycin sulfate, etc.

Chemotherapics:

- Pyridone-carboxylic acid antibacterial agents such as ofloxacin, lebofloxacin, norfloxcin, cyprofloxacin, enoxacin.
- Sulfa drugs
- Anti-viral drugs such as aciclovir.

High molecular weight substances

Peptides and proteins;

Interferons, interleukins, tumor necrosis factors, muramyl peptides, erythropoetins, epidermal growth factors (EGF), colony stimulation factors (CSF), nerve growth factors (NGF), hepatic growth factors (HGF), superoxide dismutases etc.

Nucleic acids:

DNAs such as antisense oligonucleotides,
    RNA (Peptide Nucleic Acid (PNA)).

The above-mentioned drugs are encapsulated in liposomes in a form of isotonic solution (relating to physiological osmatic pressure in the body) so that the liposomes are stably maintained in the body after administrated. As a medium, water, buffer solution such as Tris-HCℓ buffer, phosphate buffer, citrate buffer or the like may be used. To obtain an isotonic solution, a drug is dissolved in a medium at a concentration which provides an isotonic solution. In the case where a drug alone cannot provide an isotonic solution because, for example, solubility of the drug is low, other non-toxic water soluble substance, for example, salts such as sodium chloride, sugars, such as mannitol, glucose, sucrose, sorbitol, or the like may be added to the medium so that an isotonic solution is formed.

Next, a process for production of the present liposomes is explained. The present liposomes are characterized by that they are multilamellar liposomes so as to be stably maintained in the blood after administeration, and they have high encapsulation capacity. The present liposomes can be produced by conventional procedures used for formation of multilamellar liposomes. These procedures include Bangham method (J. Mol. Diol. 13, 238 - 252, 1965), polyvalent alcohol method (Japanese Examined Patent Publication (Kokoku) No. 4-36734), lipid-solution method (Japanese Examined Patent Publication (Kokoku) No. 4-36735), mechanochemical method (Japanese Examined Patent Publication (Kokoku) No. 4-28412).

Generally, desired multilamellar vesicles can be prepared by dissolving the above-mentioned phospholipids in a volatile organic solvent such as chloroform, methanol, dichloromethane, ethanol or the like, or a mixed solvent of said organic solvent and water, removing said solvent, mixing the resulting residue an aqueous phase containing a water soluble drug, and shaking or stirring the mixture.

As the step for removing solvent in the above-mentioned process, Bangham's method uses evaporation, and splay-dry method uses spray-drying. Lyophilization also can be used.

In the above-mentioned liposome-preparing processes, the amount of the solvent used relating to lipid is not limited, and in amount which allows dissolution of lipid is acceptable. Removing solvent from the resulting mixture of lipid and solvent by evaporation can be carried out according to a conventional procedure, such as evaporation under reduced pressure, if necessary in the presence of inert gas. As for solvent actually used, the above-mentioned volatile organic solvents and mixed solvents comprising 10 volumes of said organic solvent and at most 1 volume of water may be exemplified.

To remove solvent by lyophilization a solvent is selected so that the solvent can be removed at a reduced pressure of about 0.005 to 0.1 Torr at a temperature lower than the freezing point of the solvent, generally -30°C to -50°C; and the solvent is removed. To remove solvent by splay drying, generally, an air pressure is controlled to 1.0 kg/cm$^2$, an air rate is controlled to 0.35 cm$^2$/minute, and inlet temperature is adjusted to a temperature higher than the boiling point of the solvent used. For example, the solvent is chloroform, the temperature is adjusted to 60 to 90°C, and the solvent is removed according to a conventional procedure.

Lipid residue thus obtained is mixed with an aqueous solution containing a drug such as contrast agent at a temperature equal or higher than the phase transition temperature (Tc) of the lipid used, and then the mixture is vigorously or gently shaken or stirred at a temperature equal or higher than said Tc to produce desired liposomes and composition thereof.

The electrolyte ion concentration in the aqueous solution containing a drug should desirably be as low as possible in view of the encapsulation ratio of drug, and generally, the total concentration of ions including positive ions and negative ions except the drug should suitably be not more than about 40 mM more preferably not more than about 20 mM.

Although a particle size of liposome is expressed by a number average particle size, a weighted average particle size is preferably used in the point of view of encapsulation capacity.

Weight average particle size of the present liposomes is usually 50 nm to 3,000 nm, preferably 150 nm to 1000 nm, and more preferably 200 nm to 500 nm. Where the particle size is less than 200 nm, sufficient holding capacity cannot be obtained, and where the particle size is more than 3,000 nm, when the liposomes agglomerate, capillary vessels could be blocked. To obtain the above-mentioned desired particle size, liposomes having larger particle size are filtered through a filter having a predetermined pore size according to an extrusion (Biochem. Biophys. Acta Vol. 557, p9 (1979)).

The liposomes thus prepared are present as a suspension in an aqueous medium (outer liquid), and are used as a pharmaceutical composition. A solution of a drug which has not been encapsulated in liposomes during the formation of the liposomes can be present in the outer liquid. Alternatively, the solution present outside of the liposomes can be replaced with other outer liquid. In any case, an outer liquid (dispersion medium) is preferably isotonic related to the internal aqueous phase.

The electrolyte ion concentration in the liposome suspension thus prepared should desirably be as low as possible in view of the stability of liposomes on long term strage, and generally, the total concentration of ions including positive ions and negative ions except the drug should be suitably not more than about 40 mM, more preferably not more than about 20 mM. Also, the pH of the liposome suspension can be selected among the pH range wherein liposomes or the used drug can be stable, and usually it is in the range of pH 3 to 8.

Encapsulation capacity of the present liposomes is at least 5 mℓ/g lipid, preferably at least 6 mℓ/g, and more preferably 8 mℓ/g, and still more preferably at least 10 mℓ/g, and for example in the case where liposomes are prepared using a sucrose solution (280 mM) as a dispersion medium, encapsulation capacity can be more than 12 mℓ/g.

Generally, encapsulation ratio of water soluble nonelectrolytic drugs in liposomes becomes higher and higher according to increasing a concentration of liposomal lipid, however, roughly 50% is the maximum, and encapsulation ratio may not be over such maximum even if concentration of liposomal lipid would be increased.

According to the present invention, multilamellar liposomes efficiently holding a water soluble substance, especially a non-electrolytic water soluble drug in the internal aqueous phase can be obtained by a conventional method frequently used for preparation of multilamellar liposomes in spite of not so high liposomal lipid concentration. The liposomes can be heat-sterilized without coloring, change of particle size, aggregation of liposomes, or change of holding ratio, and are stable in the blood, stable during storage for more than one year, and chemically, stable as a liposome composition.

## EXAMPLES

Next, the present invention is explained in more detail by Experiments and Examples.

## Experiment

### (1) Preparation of liposomes

First, 1g of lipids was weighed and to the lipids was added 60 m$\ell$ of a mixture of chloroform, methanol and water (100:20:0.1 by volume), and the mixture was heated on a water bath (65°C) to dissolve the lipids. The solution was evaporated in a rotary evaporator at 60°C to remove the solvent.

The residue was further dried under in vacuum for 2 hours to form a lipid film. An isotonic (280 mM) aqueous solution of sucrose was heated to 65°C, and 50 m$\ell$ of the heated solution as added to the lipid film, and mixed with a mixer for 10 minutes while heating at 65°C. The mixture was then once filtered through a polycarbonate membrane filter having a pore size of 0.1 μm under a pressure to prepare multilameller vesicles (MLV).

A portion of the MLV thus prepared was put into a glass vial, and autoclaved at 121°C for 20 minutes for sterilization.

### (2) Measurement of particle size

The weight average particle size of the liposomes thus obtained was determined by a quasi-elastic light scattering method using a Dynamic light scattering meter DLS-700 (Otsuka Denshi Kabushiki Kaisha).

### (3) Measurement of encapsulation capacity

Volume of an internal aqueous phase of liposomes was calculated by a ratio of a water soluble non-electrolyte, sucrose, held in the liposomes, i.e., an encapsulation ratio of sucrose by liposome. For example, where encapsulation ratio of sucrose is 30%, 30% of 1 m$\ell$ of liposome preparation, i.e., 0.3 m$\ell$ is the volume of the internal aqueous phase. Encapsulation capacity is defined as a volume of internal aqueous phase (volume) per unit lipid (weight). For example, where concentration of lipid of a liposome preparation is 0.2 g/m$\ell$, and holding ratio of sucrose is 30%, since 0.02g of lipid carry 0.3 m$\ell$ of volume of internal aqueous phase in 1 m$\ell$ of liposomes, then encapsulation capacity is 15 m$\ell$/g.

Encapsulation ratio of sucrose in a liposome preparation was measured by a gel filtration method. Namely, liposome preparations were gel-filtered through a gel-filtration column (carrier: Pharmacia, Sephadex G50; column diameter: 16 mm; column height: 30 mm) using a physiological saline as a mobile phase, and an elute was fractionated (2.5 m$\ell$/fraction). 0.8 m$\ell$ of an aliquot was taken from each fraction, 2 m$\ell$ of methanol was added thereon, and further 1 m$\ell$ of chloroform was added. The mixture was stirred once to make the whole transparent.

1 m$\ell$ of chloroform was added thereto, and after stirring 1.2 m$\ell$ of distilled water was added to the mixture, which was then stirred and centrifuged (3500 rpm, 10 minutes, room temperature) in a cooling centrifuge (Kubota Shoji K.K., Type KR-702), and a concentration of sucrose recovered in the upper phase (water-methanol phase) of the separated two phases was measured by phenol-sulfuric acid method. In the case where a concentration of sucrose in the extract is high, the extract was appropriately diluted with distilled water, 0.8 m$\ell$ of an aliquot was taken and extracted with methanol, chloroform and distilled water.

An amount of sucrose in liposome fraction eluted in the void volume was divided by a total amount of sucrose recovered in the elute (an amount of sucrose recovered in 25 fractions) to obtain an encapsulation ratio. Note that the end point of liposome fractions was a fraction whose sucrose concentration was minimized.

### (4) Effects of lipid composition

Hydrogenated lecithin (HEPC) prepared by hydrogenating egg yolk-derived lecithin (EPC) and phosphatidylserine (HSPS) synthesized from a hydrogenated lecithin prepared by hydrogenating soybean-derived lecithin were weighed as shown in Table 1, and liposome preparations were prepared by the above-mentioned method, and after heat-sterilization, particle size and holding capacity of the liposome preparations were measured, as described above.

Table 1

| Weight of | | Particle size (nm, Mean ± S.D.) | | Encapsulation capacity (ml/g lipid) | |
|---|---|---|---|---|---|
| HEPC | HSPS | Before autoclaving | After autoclaving | Before autoclaving | After autoclaving |
| 0.985g | 0.015g | 373±179 | 349±180 | 9.8 | 9.6 |
| 0.975g | 0.025g | 265±122 | 271±128 | 15.5 | 15.5 |
| 0.950g | 0.050g | 237±111 | 260±119 | 16.0 | 15.8 |
| 0.909g | 0.091g | 260±118 | 227±102 | 13.9 | 14.3 |
| 0.850g | 0.150g | 235±115 | 241±113 | 14.1 | 13.8 |
| 0.800g | 0.200g | 197± 91 | 92± 87 | 8.9 | 8.9 |

(5) Effects of particle size

0.909g of HEPC and 0.091g of HSPC were weighed, and liposome preparations were prepared by the above-mentioned method, except that a polycarbonate membrane filter having a pore size of 1.0 $\mu$m, 0.8 $\mu$m or 0.6 $\mu$m was used for pressure filtration. Particle size and encapsulation capacity were measured before and after heat-sterization, as described above. A result is shown in Table 2.

Table 2

| Pore size of filter | Particle size (nm, Mean ± S.D.) | | Encapsulation capacity (ml/g lipid) | |
|---|---|---|---|---|
| | Before autoclaving | After autoclaving | Before autoclaving | After autoclaving |
| 1.0 $\mu$m | 260±118 | 227±102 | 13.9 | 14.3 |
| 0.8 | 225±102 | 214±102 | 14.1 | 14.2 |
| 0.6 | 213± 92 | 213± 93 | 10.8 | 10.6 |

(6) Effects of kind of charged lipids

0.950g of HEPC and 0.050g of the charged lipide shown in Table 3 were weighed, and liposome preparations were prepared by the above-mentioned method, and particle size and encapsulation capacity were measure before and after heat-sterilization.

Table 3

| Charged lipid | Particle size (nm, Mean ± S.D.) | | Encapsulation capacity (ml/g lipid) | |
|---|---|---|---|---|
| | Before heating | After heating | Before heating | After heating |
| HSPS | 237±111 | 260±119 | 16.0 | 15.8 |
| DSPG | 254±121 | 252±123 | 14.6 | 14.7 |
| DPPA | 276±123 | 239±111 | 14.8 | 14.6 |

Example 1. Encapsulation of X-ray imaging agent, iodixanol

0.640g of hydrogenated phosphatidylcholine derived from egg yolk (HEPC) and 0.064g of hydrogenated phosphatidylserine (HEPS) synthesized from HEPC were weighed, and 60 mℓ of a mixture of chloroform, methanol and water (volume ratio 100:20:0.1) was added to the weighed phospholipids.

The mixture was heated on a water bath (65°C) to dissolve the phospholipids, and the resulting solution was heated in a rotary evaporator at 60°C to evaporate the solvent. The residue was further dried in vacuum for 2 hours to form a lipid film. An aqueous solution containing iodixanol (1,3-bis(acetylamino-N,N'-bis[3,5-bis(2,3-dihydroxypropylaminocarbonyl)-2,4,6-triiodophenyl]-2-hydroxypropane) (0.4 g/mℓ) and sucrose (0.05 g/mℓ) was heated to 65°C, and 10 mℓ of the heated solution was combined with the lipid film, and the mixture was stirred with a mixer for 10 minutes while heating to 65°C. This mixture was once filtered under pressure through a polycarbonate membrane filter having a pore size of 1.0 μm to prepare multilamellar vesicles (MLV).

The MLV thus prepared was put into glass vials and autoclaved at 121°C for 20 minutes for sterilization. A result is shown in Table 4.

Note that encapsulation capacity was measured according to the method of (3) of Experiment, except that a concentration of iodixanol was measured by absorbance at 246 nm.

Table 4

|  | Before autoclaving | After autoclaving |
|---|---|---|
| Particle size (nm±S.D.) | 260±116 | 251±120 |
| Encapsulation capacity (ml/g lipid) | 6.4 | 6.5 |
| Iodine/lipid weight ratio | 1.3 | 1.3 |
| Encapsulation ratio (%) | 45.1 | 45.8 |

Example 2. Encapsulation of idixanol

Liposome formulations containing iodixanol were prepared according to the method described in Example 1. A result is shown in Table 5.

Table 5

| Concentration in formulation (mg/ml) | | | Particle size | | Holding capacity | | Iodine/lipid weight ratio | |
|---|---|---|---|---|---|---|---|---|
| HEPC | HEPS | Iodixanol | Before AC | After AC | Before AC | After AC | Before AC | After AC |
| 60.4 | 6.4 | 400 | 260±116 | 251±120 | 6.4 | 6.5 | 1.3 | 1.3 |
| 60.4 | 6.4 | 200 | 273±123 | 275±118 | 7.5 | 8.0 | 0.8 | 0.8 |
| Note: AC = autoclaving | | | | | | | | |

Example 3. Encapsulation of ouabain

5.0g of HEPC and 0.5g of HEPES were weighed into a flask, and a mixture of chloroform, methanol and water (volume ratio 100:25:0.1) was added thereon, and the components were dissolved by heating in a water bath (65°C). The solvent was distilled off on a rotary evaporator at 60°C and the residue was further dried in vacuum for 12 hours to form lipid film. On the other hand, 5.0g of HEPES and 0.5g of distealoyl phosphatidic acid (DSPA) were weighed so as to prepare a lipid film as described above. A part of the film was weighed from the flask, and used to prepared liposomes. Namely, an aqueous solution containing ouabain (5 mg/ml) and saccharose (0.09 g/ml) was heated to 65°C, and 50 ml of the ouabain aqueous solution was added to the lipid film (1g) which had been previously added in a glass beaker, and the mixture was stirred by a high speed homogenizer (Tokushu Kika Kogyo, Osaka, Japan) for about 1 minute while heating at 65°C. The mixture was filtered twice under pressure through a membrane filter made from polycarbonate resin having a pore size of 1.0 μm so as to prepare multilamellar vesicles (MLV).

Particle size and encapsulation ratio were measured according to the procedure as described in paragraphs (2) and (3) of Experiment, except that in the measured of encapsulation ratio, an amount of ouabain was determined by measuring absorbance (221 nm) of a liquid prepared by diluting 1 ml of fractions after gel filtration with 4 ml of methanol. A result is shown in Table 6.

Table 6

| Lipid composition | HEPC/HEPS (10:1) | HEPC/DSPA (10:1) |
|---|---|---|
| Particle size (nm±S.D.) | 205±101 | 208±98 |
| Encapsulation ratio | 34.2 | 45.1 |
| Encapsulation capacity (Liter/g lipid) | 13.7 | 18.0 |

Example 4. Encapsulation of sodium salicylate

A part of the lipid film prepared in Example 3 was used to prepare liposomes according to the procedure as described in Example 3. Namely an aqueous solution containing sodium salicylate (10 mg/ml) and saccharose (0.05 g/ml) was heated to 65°C, and 50 ml of the sodium salicylate aqueous solution was added to the lipid film which had been previously added to a glass beaker so as to prepare MLV as described in Example 2.

Particle size was measured by the procedure described in paragraph (2) of Experiment. Encapsulation ratio was measured by ultrafiltration method. More specifically, 0.5 ml of liposomes was put into a 50 ml calibrated flask and filled up to 50 ml with 5% glucose aqueous solution. 2.5 ml of the diluted solution (A solution) was put into outer tube of a ultrafiltration unit (Centrisart$^R$, cut-off 20000, SARTORIUS), a floater was attached thereto, and centrifugation was carried out at 3500 rpm, for 10 minutes at a room temperature. A filtrate was obtained from the floater, to 1 ml of the filtrate was added 2 ml of methanol, and absorbance at 297 mm (ABS (B)) was measured. On the other hand, 2 ml of methanol was added to 1 ml of the A solution, and absorbance at 299 nm (ABS(A)) was measured. Encapsulation ratio was calculated according to the following equation:

$$\text{Encapsulation ratio} = (ABS(A)\text{-}ABS(B))/ABS(A) \times 100\%$$

Table 7

| Lipid Composition | HEPC/HEPS(10:1) | HEPC/DSPA(10:1) |
|---|---|---|
| Particle size (nm±S.D.) | 250±75 | 253±78 |
| Encapsulation ratio | 40.3 | 41.9 |
| Encapsulation capacity (Liter/g lipid) | 16.1 | 16.7 |

Example 5. Encapsulation of levofloxacin

A part of the lipid film prepared in Example 2 was used to prepare liposomes according to the procedure described in Example 2. Namely an aqueous solution of levofloxacin (0.1 mg/ml) and saccharose (0.09 g/ml) was heated to 65°C, and 50 ml of the levofloxacin aqueous solution was added to the lipid film (1g) which had been previously weighed into a glass beaker, and liposomes was obtained according to the procedure as described in Example 2.

Particle size and encapsulation ratio were measured according to the procedure as described in paragraphs (2) and (3) of Experiment, except that in the measurement of encapsulation ratio, an amount of levofloxacin was determined by measuring a fluorescence (excitation wavelength 356 nm; fluorscence wave length 455 nm) of a liquid containing the liposome component lipides, prepared by adding 4 ml of methanol to 1 ml of each fraction after the gel filtration. Result shown in Table 8.

Table 8

| Lipid composition | HEPC/HEPS(10:1) | HEPC/DSPA(10:1) |
|---|---|---|
| Particle size (nm±S.D.) | 326±234 | 367±175 |
| Encapsulation ratio (%) | 27.6 | 24.6 |
| Encapsulation capacity (Liter/g lipid) | 11.0 | 9.8 |

<u>Example 6.</u>

10.0g of HEPC and 1.0g of HEPS were weighed to a flask, and 500 ml of a mixture of chloroform, methanol and water (volume ratio 100:25:0.1) was added thereon, and the whole was heated in a water bath (65°C) to dissolve the components. The solvents were distilled off on a rotary evaporator by heating the mixture at 60°C. The residue was further dried in vacuum for 12 hours to prepare a lipid film. A portion of the film was weighed from the flask to use it to prepare liposomes. Namely, 100 ml of an aqueous solution containing iodixanol (407 mg/ml) and D-sorbitol 8 mg/ml) was previously heated to 65°C, and the solution was added to a glass beaker containing the lipid film (5.63g), and the mixture was stirred for about 5 minutes with a high speed homomixer (Tokushu Kika Kogyo, Osaka, Japan) while heating at 65°C. The mixture was once filtered through a membrane filter made of polycarbonate and having pore size of 0.4 μm so as to prepare multilameller vesiclers, and Tris-HC$\ell$ buffer was added thereto (resulting concentration of Tris-HC$\ell$: 8 mM; resulting pH: 7.8) which were then put into a glass viales and autoclaved at 121°C for 20 minutes.

After autoclaving, the particle size and encapsulation ratio of the product were measured according to the same procedure as described in the paragraphs (2) and (3) of Experiment. A results is shown in Table 9.

Table 9

| Particle size (nm±S.D.) | 315±157 |
|---|---|
| Encapsulation ratio (%) | 38.4 |
| Encapsulation volume (Liter/g lipid) | 6.82 |
| Weight ratio of iodine/lipid | 1.36 |

**Claims**

1. Liposomes and suspensions thereof comprising multilamellar liposomes characterized in that the membranes of liposomes are formed from a neutral phospholipid comprising a substantially saturated fatty acid residue and a charged phospholipid comprising a substantially saturated fatty acid residue, weight ratio of said neutral phospholipid and said charged phospholipid is 200:1 to 3:1, average particle size of the liposomes is 50 nm to 3,000 nm, and a holding capacity of the liposome (internal aqueous phase (volume)/lipids (weight)) is at least 5 m$\ell$/g.

2. Liposomes and suspensions thereof according to claim 1, wherein the number of fatty acid residue of the neutral phospholipid and charged phospholipid is at least 14.

3. Liposomes and suspensions thereof according to claim 2, wherein the neutral lipid is a phosphatidylcholine.

4. Liposomes and suspensions thereof according to claim 3, wherein the phosphatidylcholine is dipalmitoyl phosphatidylcholine or distearoyl phosphatidylcholine.

5. Liposomes and suspensions thereof according to claim 2, wherein the charged phospholipid is 1 phosphatidylserine, phosphatidylglycerol, phosphatidyllinositol, phosphatidic acid or an ester of phosphatidic acid and an aminoalcohol.

6. Liposomes and suspensions thereof according to claim 5, wherein the phosphatidylserine is dipalmitoyl phosphatidylserine or distearoyl phosphatidylserine.

7. Liposomes and suspensions thereof according to claim 5, wherein the phosphatidylglycerol is dipalmitoyl phosphatidylglycerol or distearoyl phosphatidylglycerol.

8. Liposomes and suspensions thereof according to claim 5, wherein the phosphatidylinositol is dipalmitoyl phosphatidylinositol or distearoyl phosphatidylinositol.

9. Liposomes and suspensions thereof according to claim 5, wherein the phosphatidic acid is dipalmitoyl phosphatidic acid or distearoyl phosphatidic acid.

10. Liposomes and suspensions thereof according to claim 5, wherein the ester of a phosphatidic acid and an aminoalcohol is an ester of dipalmitoyl phosphatidic acid or distearoyl phosphatidic acid and hydroxyethylene diamine.

11. Liposomes and suspensions thereof according to any one of claims 1 to 10, wherein the weight ratio of the neutral phospholipid and the charged phospholipid is 60:1 to 4:1.

12. Liposomes and suspensions thereof according to any one of claims 1 to 11, wherein the average particle size of the liposomes is 150 nm to 1000 nm.

13. Liposomes and suspensions thereof according to any one of claims 1 to 11, wherein the encapsulation capacity is at least 6 m$\ell$/g.

14. Liposomes and suspensions thereof according to any one of claims 1 to 11, wherein the encapsulation capacity is at least 8 m$\ell$/g.

15. Liposomes and suspensions thereof according to any one of claims 1 to 11, wherein the encapsulation capacity is at least 10 m$\ell$/g.

16. Liposomes and suspensions thereof according to any one of claims 1 to 15, wherein the concentration of total lipid is 20 mg/m$\ell$ to 100 mg/m$\ell$.

17. Liposomes and suspensions thereof according to any one of claims 1 to 15, wherein the concentration of total lipid is 40 mg/m$\ell$ to 90 mg/m$\ell$.

18. Liposomes and suspensions thereof according to any one of claims 1 to 15, wherein the concentration of total lipid is 50 mg/m$\ell$ to 80 mg/m$\ell$.

19. Liposomes and suspensions thereof according to any one of claims 1 to 18, wherein a water soluble drug is encapsulated in an inner aqueous phase of the liposome.

20. Liposomes and suspensions thereof according to any one of claims 1 to 19, wherein a water soluble and nonelectrolytic drug is encapsulated in an inner aqueous phase of the liposomes.

21. Liposomes and suspensions thereof according to any one of claims 1 to 19, wherein a water soluble and oppositely charged drug to the charged phospholipid is encapsulated in an inner aqueous phase of the liposomes.

22. A process for production of liposomes and suspensions thereof characterized by dissolving a neutral phospholipid containing substantially saturated fatty acid residue and charged phospholipid containing substantially saturated fatty acid residue in a solvent, removing the solvent to obtain a residue, and mixing the residue of an aqueous solution which may contain a drug, and shaking or stirring.

## INTERNATIONAL SEARCH REPORT

| International application No. |
| --- |
| PCT/JP95/00566 |

**A. CLASSIFICATION OF SUBJECT MATTER**

Int. Cl$^6$ A61K9/127, 47/24, B01J13/02

According to International Patent Classification (IPC) or to both national classification and IPC

**B. FIELDS SEARCHED**

Minimum documentation searched (classification system followed by classification symbols)

Int. Cl$^6$ A61K9/127, 47/24, B01J13/02

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>A | JP, 5-194192, A (Shionogi & Co., Ltd.),<br>August 3, 1993 (03. 08. 93)(Family: none)<br>Line 20, column 4 to line 13, column 6 | 22<br>1 - 21 |
| X<br><br>A | JP, 3-63226, A (Kyowa Hakko Kogyo Co., Ltd.),<br>March 19, 1991 (19. 03. 91)(Family: none)<br>Line 9, upper left column to last line, upper<br>right column, page 3 | 22<br><br>1 - 21 |

☐ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | | |
| "O" | document referring to an oral disclosure, use, exhibition or other means | "Y" | document of particular relevance; the claimed invention cannot be considered to involve an inventive step when the document is combined with one or more other such documents, such combination being obvious to a person skilled in the art |
| "P" | document published prior to the international filing date but later than the priority date claimed | "&" | document member of the same patent family |

| Date of the actual completion of the international search | Date of mailing of the international search report |
| --- | --- |
| June 12, 1995 (12. 06. 95) | July 4, 1995 (04. 07. 95) |

| Name and mailing address of the ISA/ | Authorized officer |
| --- | --- |
| Japanese Patent Office | |
| Facsimile No. | Telephone No. |

Form PCT/ISA/210 (second sheet) (July 1992)